# EUROPÄISCHE PATENTANMELDUNG

(11) **EP 3 791 783 A1**
(43) Veröffentlichungstag der Anmeldung: **17.03.2021**
(21) Anmeldenummer: 19196488.1
(22) Anmeldetag: 10.09.2019
(51) Int. Cl.: A61B 5/042, A61B 5/00, A61M 25/00, A61B 18/14

(54) **KATHETER MIT DEHNBARER LEITERPLATTE**

(71) Anmelder: Carl Freudenberg KG, 69469 Weinheim (DE)
(72) Erfinder: Schmied, Benno, 67071 Ludwigshafen (DE)

(57) **Zusammenfassung**

Die Erfindung betrifft einen Katheter (10) in Form eines länglichen, flexiblen Rohres (1) mit mindestens einer Elektrode (2) zur Durchführung einer Diagnose oder Therapie im menschlichen Körper, wobei die mindestens eine Elektrode (2) an einem Endbereich des Rohres (1) angeordnet ist und am anderen Endbereich des Rohres (1) ein elektrischer Anschluss (3) angebracht ist zum elektrischen Verbinden des Katheters (10) mit einer Steuereinrichtung (8). Erfindungsgemäß sind die mindestens eine Elektrode (2) und der elektrische Anschluss (3) mittels Leiterbahnen (5) auf einer dehnbaren, elastischen Leiterplatte (4), insbesondere aus thermoplastischen Polymer, elektrisch leitend miteinander verbunden und die dehnbare Leiterplatte (4) bildet die Mantelfläche des Katheters (10).

Durch die Erfindung wird so ein kostengünstiger und gleichzeitig sehr flexibler Katheter geschaffen.

## Beschreibung

Die Erfindung betrifft einen Katheter gemäß dem Oberbegriff von Anspruch 1.

### Stand der Technik

Aus dem Stand der Technik sind unterschiedlichste Bauformen von Kathetern bekannt, welche ihrer medizinischen Anwendung entsprechend aufgebaut sind. Darunter sind auch elektrodentragende Katheter wie sie beispielsweise in der DE 694 01 562 T2 beschrieben sind. Auch bekannt sind sogenannte Mappingkatheter, beispielsweise zur 3-dimensionalen Erfassung der Anatomie einer Herzkammer, welche eine hohe Anzahl an Elektroden aufweisen müssen.

Bei den aus dem Stand der Technik bekannten Kathetern entstehen hohe Produktionskosten, weil die zu den Elektroden führenden Leiterbahnen aufwändig im Katheterrohr untergebracht werden müssen. Weiter problematisch ist, dass sich auf Grund der verwendeten Materialien eine Beschränkung bzgl. des Biegeradius des Katheters ergeben kann und damit eine Einschränkung in dessen Einsatz.

### Aufgabenstellung

Aufgabe der vorliegenden Erfindung ist es einen kostengünstigen und gleichzeitig flexiblen Katheter zu schaffen und die Nachteile des Standes der Technik zumindest teilweise zu beheben.

### Technische Lösung

Gelöst wird diese Aufgabe durch einen Katheter mit einer dehnbaren Leiterplatte mit den Merkmalen von Anspruch 1.

Erfindungsgemäß wurde als vorteilhaft erkannt dehnbare Leiterplatten einzusetzen:
Der Katheter hat die Form eines länglichen, flexiblen Rohres welches auch als Schlauch bezeichnet werden könnte. Der Katheter besitzt mindestens eine Elektrode zur Durchführung einer Diagnose, beispielsweise einer Messung wie einer elektrophysiologischen Untersuchung, oder zur Durchführung einer Therapie, beispielsweise einer Operation wie einer Katheterablation, jeweils im menschlichen Körper. Die mindestens eine Elektrode ist an einem Endbereich des Rohres angeordnet und am anderen Endbereich des Rohres ist ein elektrischer Anschluss angebracht zum elektrischen Verbinden des Katheters mit einer Steuereinrichtung, sodass eine stromleitende und datenübertragungstechnische Verbindung zwischen der mindestens eine Elektrode und dem elektrischen Anschluss besteht.

In vorteilhafter Weise sind die mindestens eine Elektrode und der elektrische Anschluss mittels Leiterbahnen auf mindestens einer dehnbaren Leiterplatte, d. h. einer elastischen Leiterplatte, elektrisch leitend miteinander verbunden und die dehnbare Leiterplatte bildet die Mantelfläche des Katheters. Ein solcher Katheter besitzt eine sehr gute Flexibilität und kann für verschiedenste Diagnosen und Therapien eingesetzt werden.

In einer ersten Variante kann die Leiterplatte dazu auf der Mantelfläche des Rohres angeordnet sein. Das Rohr kann dazu beispielsweise von der Leiterplatte umwickelt werden. Besonders vorteilhaft ist es wenn sich die Leiterplatte über den gesamten Umfang der Mantelfläche des Rohres erstreckt. Dann verbleiben in der Oberfläche der Mantelfläche keine Unebenheiten. In einer zweiten Variante wird das Rohr durch die Leiterplatte ausgebildet. Dies hat den Vorteil, dass der Katheter aus möglichst wenigen Materialien in wenigen Arbeitsschritten gefertigt werden kann.

In vorteilhafter Weiterbildung des erfindungsgemäßen Katheters ist die Leiterplatte mit Leiterbahnen aus Kupfer oder Kupferlegierungen versehen. Diese können insbesondere in einem subtraktiven Verfahren aufgebracht sein. Ein solcher Katheter kann besonders schnell und kostengünstig gefertigt werden. Die Endbereiche der Leiterbahnen, die sogenannten Pads, werden hierbei nicht als Teil der Leiterbahnen angesehen. Die Pads, welche zum Beispiel als Elektronen dienen, können auch andere Legierungen, wie zum Beispiel Silberlegierungen aufweisen.

Es hat sich als besonders vorteilhaft erwiesen wenn die Leiterbahnen eine Mäanderform aufweisen, insbesondere eine rechteckige, wellenförmige, u-förmige oder hufeisenförmige Mäandrierung (Mäander). Derartige Mäandrierungen (Mäander) werden gebildet durch Aneinanderreihung von rechteckigen, wellenförmigen, u- förmigen oder hufeisenförmigen Mäandergrundformen.

In Versuchen hat sich gezeigt, dass es besonders vorteilhaft ist, wenn sich die Mäandergrundformen der Mäanderform mit ihren zwischen den Krümmungen bzw. Ecken gelegenen längeren Schenkeln jeweils über einen Großteil des Umfangs der Mantelfläche des Rohres erstrecken, insbesondere über mehr als 30%, besonders bevorzugt über 60 bis 80%. Die Beanspruchung der Leiterbahnen beim Biegen des Rohres im Einsatz des Katheters können so in einem zulässigen Bereich gehalten werden.

In einer besonders vorteilhaften und daher bevorzugten Weiterbildung des erfindungsgemäßen Katheters besteht die Leiterplatte aus einem thermoplastischen Polymer, beispielsweise aus Polyurethan (PU), insbesondere aus einem hochelastischen, thermoplastischen Polyurethan (TPU). Diese Materialien sind besonders gut dehnbar, sodass sich besonders flexible Katheter ergeben.

Die Erfindung betrifft auch die Verwendung eines wie obenstehend beschriebenen Katheters als Herzkatheter, welcher in der Diagnose oder Therapie des menschlichen Herzens zum Einsatz gelangt.

Die beschriebene Erfindung und die beschriebenen vorteilhaften Weiterbildungen der Erfindung stellen auch in Kombination miteinander - soweit dies technisch sinnvoll ist - vorteilhafte Weiterbildungen der Erfindung dar.

Hinsichtlich weiterer Vorteile und in konstruktiver und funktioneller Hinsicht vorteilhafter Ausgestaltungen der Erfindung wird auf die Unteransprüche sowie die Beschreibung von Ausführungsbeispielen unter Bezugnahme auf die beiliegenden Figuren verwiesen.

### Ausführungsbeispiel

Die Erfindung soll an Hand beigefügter Figuren noch näher erläutert werden. Einander entsprechende Elemente und Bauteile sind in den Figuren mit gleichen Bezugszeichen versehen. Zugunsten einer besseren Übersichtlichkeit der Figuren wurde auf eine maßstabsgetreue Darstellung verzichtet.
Es zeigen in schematischer Darstellung
- Fig. 1: eine dehnbaren Leiterplatte bevor und nachdem sie zu einem Rohr geformt wurde
- Fig. 2: einen erfindungsgemäßen Katheter
- Fig. 3: unterschiedliche Mäanderformen der Leiterbahnen

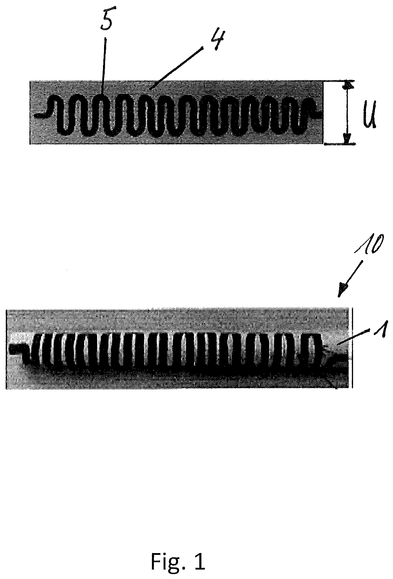

Fig. 1 zeigt eine dehnbaren Leiterplatte 4 bevor und nachdem sie zu einem Rohr 1 eines Katheters 10 geformt wurde. Auf der Leiterplatte 4 befinden sich Leiterbahnen 5, welche eine Mäanderform aufweisen. Die Mäandergrundformen der Leiterbahnen 5 erstrecken sich über einen Großteil des Umfangs U der Mantelfläche des Rohres 1.

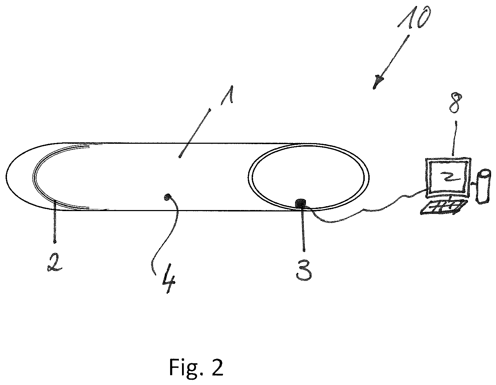

Fig. 2 zeigt einen erfindungsgemäßen Katheter 10, welcher die Form eines länglichen, flexiblen Rohres 1 besitzt. An seinem einen Endbereich ist mindestens eine Elektrode 2 angeordnet. An seinem anderen Endbereich ist ein elektrischer Anschluss 3 angebracht. Die mindestens eine Elektrode 2 ist über Leiterbahnen 5 auf einer elastisch dehnbaren Leiterplatte 4 (hier nicht näher dargestellt, siehe Figur 1) mit dem elektrischen Anschluss 3 datenübertragungstechnisch und stromleitend verbunden. An den Anschluss 3 ist eine Steuereinrichtung 8 angeschlossen, welche zur Steuerung des Katheters 10 dient.

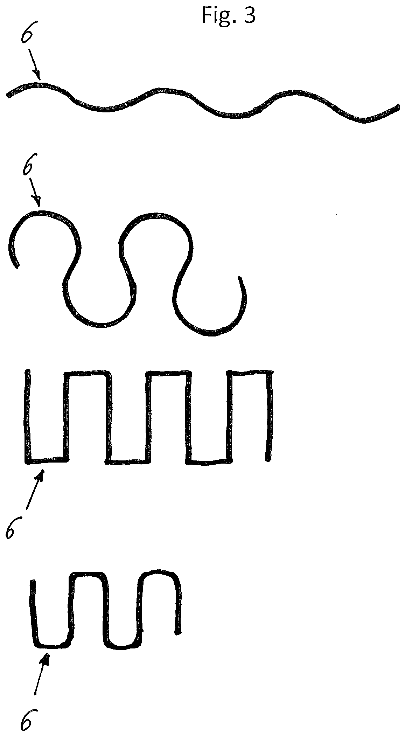

Fig. 3 zeigt unterschiedliche Mäanderformen, welche die Leiterbahnen 5 auf der elastisch dehnbaren Leiterplatte 4 aufweisen können: die Mäandergrundform 6 kann dabei wellenförmig, hufeisenförmig, rechteckig oder u-förmig sein.

### Bezugszeichenliste

- 1: Rohr
- 2: Elektrode
- 3: Anschluss
- 4: Leiterplatte
- 5: Leiterbahn
- 6: Mäandergrundform
- 7: -
- 8: Steuereinrichtung
- 9: -
- 10: Katheter

- U: Umfang der Mantelfläche des Rohres

## Patentansprüche

1. Katheter (10) in Form eines länglichen, flexiblen Rohres (1) mit mindestens einer Elektrode (2) zur Durchführung einer Diagnose oder Therapie im menschlichen Körper, wobei die mindestens eine Elektrode (2) an einem Endbereich des Rohres (1) angeordnet ist und am anderen Endbereich des Rohres (1) ein elektrischer Anschluss (3) angebracht ist zum elektrischen Verbinden des Katheters (10) mit einer Steuereinrichtung (8)
**dadurch gekennzeichnet, dass**
die mindestens eine Elektrode (2) und der elektrische Anschluss (3) mittels Leiterbahnen (5) auf einer dehnbaren Leiterplatte (4) elektrisch leitend miteinander verbunden sind und
die dehnbare Leiterplatte (4) die Mantelfläche des Katheters (10) bildet.

2. Katheter nach Anspruch 1 **dadurch gekennzeichnet, dass**
die Leiterplatte (4) auf der Mantelfläche des Rohres (1) angeordnet ist.

3. Katheter nach Anspruch 2, **dadurch gekennzeichnet, dass**
sich die Leiterplatte (4) über den gesamten Umfang (U) der Mantelfläche des Rohres (1) erstreckt.

4. Katheter nach Anspruch 1, **dadurch gekennzeichnet, dass**
die Leiterplatte (4) das Rohr (1) ausbildet.

5. Katheter nach einem der vorangehenden Ansprüche, **dadurch gekennzeichnet, dass**
die Leiterplatte (4) mit Leiterbahnen (5) aus Kupfer oder Kupferlegierungen versehen ist.

6. Katheter nach einem der vorangehenden Ansprüche, **dadurch gekennzeichnet, dass**
die Leiterbahnen (5) eine Mäanderform aufweisen, insbesondere eine rechteckige, wellenförmige, u-förmige oder hufeisenförmige Mäandrierung.

7. Katheter nach Anspruch 6, **dadurch gekennzeichnet, dass**
sich die Mäandergrundformen (6) mit ihren zwischen den Krümmungen bzw. Ecken gelegenen längeren Schenkeln jeweils über einen Großteil des Umfangs (U) der Mantelfläche des Rohres (1) erstrecken, insbesondere über mehr als 30%, besonders bevorzugt über 60 bis 80%.

8. Katheter nach einem der vorangehenden Ansprüche, **dadurch gekennzeichnet, dass**
die Leiterplatte (4) aus einem thermoplastischen Polymer besteht, beispielsweise aus Polyurethan, insbesondere aus einem hochelastischen, thermoplastischen Polyurethan.
